# EUROPEAN PATENT APPLICATION

(11) **EP 4 835 903 A1**
(43) Date of publication of application: **04.11.2026**
(21) Application number: 25173965.2
(22) Date of filing: 02.05.2025
(51) Int. Cl.: G16H 40/63, G16H 30/40, A61B 34/20, A61B 34/00, A61B 90/00, G06F 3/16, A61B 90/20, A61B 17/00

(54) **SYSTEM, METHOD AND COMPUTER PROGRAM FOR A SURGICAL IMAGING SYSTEM, SURGICAL IMAGING SYSTEM**

(71) Applicant: Leica Instruments (Singapore) Pte Ltd., Singapore 618299 (SG)
(72) Inventor: ZHANG, Charles, 618299 Singapore (SG); ANG, Yan Eng, 618299 Singapore (SG)
(74) Representative: 2SPL Patentanwälte PartG mbB

(57) **Abstract**

Examples relate to a system (110), a method, and a computer program for a surgical imaging system (100), and to a surgical imaging system (100) comprising such a system (110). The system (110) is configured to obtain a stream of image data of an imaging device (120, 150) of the surgical imaging system, process the stream of image data to track a position of a portion of a surgical tool, determine, based on a change in the position of the portion of the surgical tool, a position of a digital pointing indicator relative to a field of view of a surgical imaging device (120) of the surgical imaging system showing a surgical site, generate an overlay comprising the digital pointing indicator to be overlaid over the field of view of the surgical imaging device, provide the overlay with the digital pointing indicator for a display device (130a, 130b) of the surgical imaging system, obtain a voice command of a user of the surgical imaging system, the voice command comprising an instruction to perform a spatially localized functionality, and trigger performing the spatially localized functionality according to the position of the digital pointing indicator relative to the field of view of the surgical imaging device based on the voice command.

## Description

### Technical field

Examples relate to a system, a method, and a computer program for a surgical imaging system, and to a surgical imaging system comprising such a system.

### Background

Currently, surgical microscope systems require surgeons to manually control focus and other functions. Surgeons usually use additional tools for this purpose, such as foot pedals, touch screens, or even mouth controllers. This process demands significant attention and coordination, which detracts from the surgeon's ability to focus on the surgery itself. The process may be considered cumbersome and may interrupt the surgical workflow, as surgeons must frequently navigate the entire image and make iterative adjustments to achieve optimal focus. Additionally, selecting specific areas for operations, such as Optical Coherence Tomography (OCT) scans, involves using the same or similar manual controls. Surgeons use them to position the cursor and confirm actions, which may increase the complexity and reduce efficiency during procedures.

There may be a desire to provide an improved concept for controlling a surgical imaging system.

### Summary

This desire is addressed by the subject matter of the independent claims.

Various examples of the present disclosure are based on the finding that, during surgery, a surgeon is necessarily in close proximity to the surgical site. While operating on the surgical site, the surgeon is equipped with a surgical tool, such as a scalpel, forceps, or a suction device. Such instruments, in particular if they have an elongated shape, can not only be used to perform surgery but also to point at different features within the surgical site. In the proposed concept, this mechanism is used to control a cursor that is overlaid over the digital view of the surgical site, enabling the surgeon to intuitively place the cursor at a location within the surgical site at which a spatially localized functionality, such as a focusing operation or an OCT scan, is to be performed. Using a voice command, the spatially localized functionality can then be triggered at the position corresponding to the cursor. Thus, the spatially localized functionality can be triggered without requiring a foot pedal, touch screen, or a mouth controller, without the surgeon being distracted from the surgical procedure.

Some aspects of the present disclosure relate to a system for a surgical imaging system. The system comprises one or more processors and one or more storage devices. The system is configured to obtain a stream of image data of (e.g., from) an imaging device of the surgical imaging system. The system is configured to process the stream of image data to track a position of a portion of a surgical tool. The system is configured to determine, based on a change in the position of the portion of the surgical tool, a position of a digital pointing indicator relative to a field of view of a surgical imaging device of the surgical imaging system showing a surgical site. The system is configured to generate an overlay comprising the digital pointing indicator to be overlaid over the field of view of the surgical imaging device. The system is configured to provide the overlay with the digital pointing indicator for a display device of the surgical imaging system. The system is configured to obtain a voice command of (e.g., from) a user of the surgical imaging system, with the voice command comprising an instruction to perform a spatially localized functionality. The system is configured to trigger performing the spatially localized functionality according to the position of the digital pointing indicator relative to the field of view of the surgical imaging device based on the voice command. This way, the spatially localized functionality can be triggered without requiring a foot pedal, touch screen, or a mouth controller, without the surgeon being distracted from the surgical procedure.

In general, the proposed concept is applicable to both fully digital surgical imaging devices (e.g., surgical microscopes or surgical exoscopes) and hybrid surgical imaging devices. In a fully digital surgical imaging device, or in a hybrid surgical imaging device operating in a fully digital mode, a view of the surgical site is generated using image data of the surgical imaging device. Accordingly, the system may be configured to obtain the image data of the surgical imaging device and to generate a view of the surgical site overlaid with the overlay and provide the view of the surgical site with the overlay to the display device of the surgical imaging system. This way, the overlay can be provided with the digital view of the surgical site, which may provide additional degrees of freedom with respect to the display devices being used as compared to a hybrid microscope, in which the overlay is overlaid over an optical view of the surgical site.

In many cases, for example, in the case of fully digital or hybrid surgical imaging devices, the view of the surgical site is or can be generated using image data of the surgical imaging device. This image data can also be used to track the surgical tool. Accordingly, the system may be configured to obtain the stream of image data from the surgical imaging device of the surgical imaging system. In this way, no additional hardware (e.g., a camera) may be necessary for tracking the surgical tool.

Alternatively, or additionally, the system may be configured to obtain the stream of image data from an overview camera of the surgical imaging system. Thus, a larger field of view can be tracked, at the expense of an additional camera.

During surgery, there are a variety of different operations performed by the surgical imaging system that are spatially localized. For example, the surgical imaging system may perform localized illumination of a specific region of the surgical site, focus at a specific point of the surgical site, capture an image of a region of interest of the surgical site, set a digital marker to which the field of view returns upon a command, or perform a scan using a measurement tool, such as an OCT scanner. Accordingly, the spatially localized functionality may be one of a focusing functionality, a functionality for setting a marker, a functionality for illuminating a portion of the surgical site/field of view, a functionality for changing the field of view, or a functionality for performing a measurement using a measurement device. The proposed concept is applicable to such spatially localized functionalities.

While some spatially localized functionalities, such as focusing, setting a marker, setting a new center point for the field of view, or performing a measurement, are to be performed at a point of the surgical site/field of view, other spatially localized functionalities, such as illumination of a portion of the surgical site or defining an extent of a new field of view, are performed on an area of the surgical site/field of view. In the proposed concept, an area can be selected by setting two points (defining two corners of the area), and using the orientation of the field of view to deduce the remaining corners of the area. For example, the system may be configured to, if the voice command indicates that the spatially localized functionality is to be performed on an area, determine two positions of the digital pointing indicator relative to the field of view of a surgical imaging device of the surgical imaging system. The system may be configured to determine the area based on the two positions and based on an orientation of the field of view of the surgical imaging device. The system may be configured to trigger performing the spatially localized functionality on the area. This way, area-based spatially localized functionalities can intuitively be triggered by the surgeon.

While it is possible for the surgeon to select positions within the field of view by pointing explicitly at them, this may be less than ideal in scenarios in which additional surgical staff or tools block some portion of the surgical site from access by the surgeon. Therefore, the digital pointing indicator may be moved by the movement of the portion of the surgical tool, but not necessarily to the position at which the portion of the surgical tool is indicated. In other words, the system may be configured to determine the position of the digital pointing indicator based on the relative movement of the portion of the surgical tool. In particular, the digital pointing indicator may be offset from the portion of the surgical tool, such that the surgeon can control the digital pointing indicator more akin to using a mouse or trackpad, and less akin to using a tablet computer. For example, the system may be configured to determine the position of the digital pointing indicator so that the digital pointing indicator is offset from the portion of the surgical tool, as perceived by a user viewing the digital pointing indicator overlaid over the view on the surgical site by the surgical imaging device. This way, there is less likelihood of the surgeon being constrained by objects blocking the surgeon's access to the entire surgical site, and smaller movements may suffice to move the digital pointing indicator to the desired position, improving the ergonomic properties of the targeting.

During surgical procedures, many if not all spatially localized functionalities are performed towards the center of the field of view of the surgical imaging device. Therefore, to avoid having to move the digital pointing indicator across the entire field of view, it may be constrained to a central portion of the field of view. In other words, the system may be configured to determine the position of the digital pointing indicator so that the digital pointing indicator is constrained to a central portion of the field of view of the surgical imaging device. For example, the central portion of the field of view may cover at least 30% (or at least 40%, or at least 50% and/or at most 90% (and/or at most 80%, at most 70%) of the area of the field of view of the surgical imaging device. This can facilitate the placement of the digital pointing indicator.

In particular, the system may be configured to determine the position of the digital pointing indicator so that the digital pointing indicator is constrained to a rectangular portion of the field of view at the center of the field of view of the surgical imaging device. A rectangular portion of the field of view is easy for the surgeon to understand, as the surgeon is, in many cases, also provided with a rectangular view of the surgical site, at least when the user uses an auxiliary screen.

In many cases, multiple surgical tools may be (partially) visible in the field of view at once. To determine which of the surgical tools is to be used for moving the digital pointing indicator, the surgeon can identify the surgical tool to be used by moving (e.g., shaking) the surgical tool after activating the digital pointing indicator. In other words, the system may be configured to, if multiple surgical tools are visible in the field of view of the surgical imaging device, determine the surgical tool being used to control the digital pointing indicator based on a movement of the surgical tool after activation of the digital pointing indicator. For example, the system may be configured to provide a visual or audio feedback after determining the surgical tool being used to control the digital pointing indicator. This way, the proposed concept can be used even at "busy" surgical sites.

More generally, providing feedback on the functionality being performed may increase the confidence of the surgeon using the system, thus making it more likely that the surgeon continues using the system. For example, the system may be configured to, after processing the voice command, include a visual indicator representing the voice command in the overlay. In this way, the surgeon can be confident that the voice command has been registered and properly interpreted.

In a surgical setting, many different types of surgical tools may be used. Most of these tools, such as scalpels, retractors, forceps, and suction tubes, can intuitively be used to point at things. For example, the system may be configured to determine the position of one of a tip (e.g., of a retractor or forceps), a blade (e.g., of a scalpel), or a tube opening (e.g., of a surgical aspirator or suction device) of the surgical tool.

Another aspect of the present disclosure relates to a surgical imaging system that comprises the system. For example, the surgical imaging system may be a surgical imaging system for use in ophthalmic surgery.

Another aspect of the present disclosure relates to a method for a surgical imaging system. The method comprises obtaining a stream of image data of an imaging device of the surgical imaging system. The method comprises processing the stream of image data to track a position of a portion of a surgical tool. The method comprises determining, based on a change in the position of the portion of the surgical tool, a position of a digital pointing indicator relative to a field of view of a surgical imaging device of the surgical imaging system showing a surgical site. The method comprises generating an overlay comprising the digital pointing indicator to be overlaid over the field of view of the surgical imaging device. The method comprises providing the overlay with the digital pointing indicator for a display device of the surgical imaging system. The method comprises obtaining a voice command of a user of the surgical imaging system. The voice command comprises an instruction to perform a spatially localized functionality. The method comprises triggering performing the spatially localized functionality according to the position of the digital pointing indicator relative to the field of view of the surgical imaging device based on the voice command.

An aspect of the present disclosure relates to a computer program that has a program code for performing the above method when the program is executed on a processor.

### Short description of the Figures

Some examples of apparatuses and/or methods will be described in the following by way of example only, and with reference to the accompanying figures, in which
- Fig. 1a: shows a block diagram of an example of a system for a surgical imaging system;
- Fig. 1b: shows a schematic diagram of an example of a surgical imaging system;
- Figs. 1c and 1d: show examples of an overlay comprising a digital pointing indicator superimposed on a view of a surgical site;
- Fig. 2: shows a flow chart of an example of a method for a surgical imaging system; and
- Fig. 3: shows a schematic diagram of an example of a system comprising an imaging device and a computer system.

### Detailed Description

Various examples will now be described more fully with reference to the accompanying drawings in which some examples are illustrated. In the figures, the thicknesses of lines, layers and/or regions may be exaggerated for clarity.

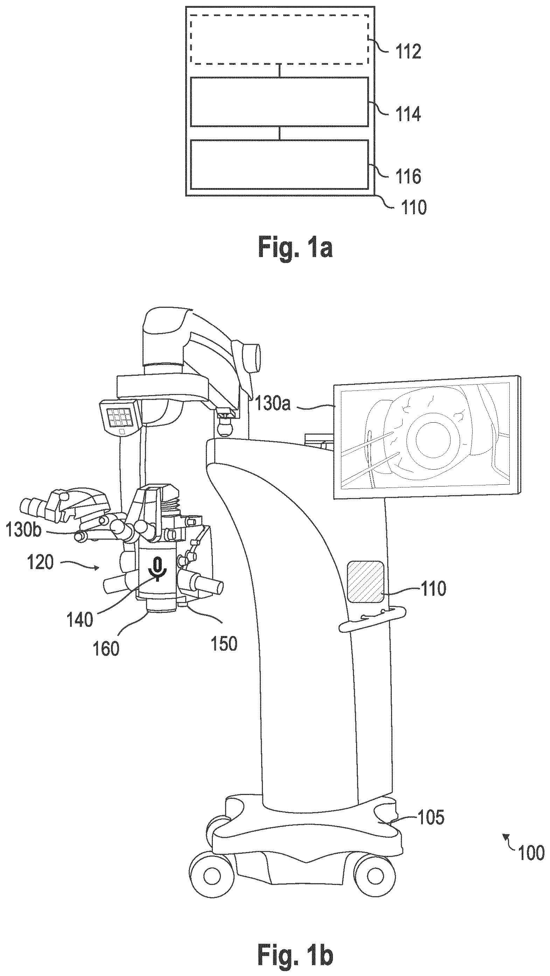

Fig. 1a shows a schematic diagram of an example of a system 110 for a surgical imaging system 100 (shown in Fig. 1b). The system 110 is a component of the surgical imaging system 100 and may be used to control various aspects of the surgical imaging system 100. In particular, it may be used for the acquisition and/or processing of imaging data by at least one optical imaging sensor of an imaging device 120 of the surgical imaging system, through various means that will be introduced in more detail in the following. In addition, the system 110 may be configured to control additional aspects of the surgical imaging system 100, for example, to provide a display signal for one or more display devices 130a, 130b of the surgical imaging system, and/or to control and/or execute automatic operations of the surgical imaging system 100, such as spatially localized functionalities provided by the surgical imaging system 100.

In general, the system 110 may be a computer system. The system 110 comprises one or more processors 114 and one or more storage devices 116. Optionally, the system 110 further comprises one or more interfaces 112. The one or more processors 114 are coupled to the one or more storage devices 116 and to the one or more interfaces 112. In general, the functionality of the system 110 may be provided by the one or more processors 114, in conjunction with the one or more interfaces 112 (for exchanging data/information with one or more other components of the surgical imaging system 100 and outside the surgical imaging system 100, such as an optical imaging sensor of the imaging device 120, one or more display devices 130a, 130b, a microphone 140, an overview camera 150 and/or an OCT scanner 160), and with the one or more storage devices 116 (for storing information, such as machine-readable instructions of a computer program being executed by the one or more processors). In general, the functionality of the one or more processors 114 may be implemented by the one or more processors 114 executing machine-readable instructions. Accordingly, any feature ascribed to the one or more processors 114 may be defined by one or more instructions of a plurality of machine-readable instructions. The system 110 may comprise the machine-readable instructions, e.g., within the one or more storage devices 116.

As outlined above, the system 110 is part of the surgical imaging system 100, which comprises various components in addition to the system 110. For example, the surgical imaging system 100 comprises the imaging device 120 and may comprise one or more additional components, such as one or more display devices 130a, 130b, such as ocular displays 130b and/or an auxiliary display 130a. Fig. 1b shows a schematic diagram of an example of such a surgical imaging system 100, and in particular of an ophthalmic surgical microscope system 100. A surgical microscope system is a surgical imaging system 100 that comprises a (surgical) microscope as the imaging device 120. However, the proposed concept is not limited to such embodiments. The surgical imaging system 100 may be based on various (single or multiple) imaging devices, such as one or more microscopes, one or more endoscopes, and/or one or more exoscopes (also sometimes called an extracorporeal telescope). Exoscopes are camera-based imaging systems, and in particular camera-based 3D imaging systems, which are suitable for providing images of surgical sites with high magnification and a large depth of field. Compared to microscopes, which may be used via oculars 130a, exoscopes are only used via display modalities, such as a monitor or a head-mounted display. Accordingly, the surgical imaging system 100 may alternatively be a surgical endoscope system or a surgical exoscope system. The following illustrations assume that the surgical imaging device 120 is a surgical microscope or surgical exoscope, and that the surgical imaging system 100 is a surgical microscope system 100 or surgical exoscope system 100.

Accordingly, the surgical imaging system or surgical microscope system 100 may comprise an imaging device, such as a microscope 120. In general, a microscope, such as the microscope 120, is an optical instrument that is suitable for examining objects that are too small to be examined by the human eye (alone). For example, a microscope may provide optical magnification of a sample, such as a surgical site. In the present concept, the optical magnification is (also) provided for at least one optical imaging sensor. The microscope 120 thus comprises an optical imaging sensor, which is coupled with the system 110. The microscope 120 may further comprise one or more optical magnification components that are used to magnify a view of the sample, such as an objective (i.e., lens). For example, the surgical imaging device or microscope 120 is often referred to as the 'optics carrier' of the imaging system.

There are a variety of different types of surgical imaging devices. If the imaging device is used in the medical or biological fields, the object being viewed through the imaging device may be a sample of organic tissue, for example, arranged within a petri dish or present in a part of a body of a patient. In various examples presented here, the imaging device 120 may be a microscope of a surgical microscope system, that is, a microscope that is to be used during a surgical procedure, such as an ophthalmic procedure (i.e., eye surgery). Accordingly, the sample being viewed through the surgical imaging device may be a sample of organic tissue of a patient and may, in particular, be the surgical site that the surgeon operates on during the surgical procedure, for example, the eye. However, the proposed concept is also suitable for other types of surgery, such as brain surgery or cardiac surgery.

Fig. 1b shows a schematic diagram of an example of a surgical imaging system 100, and in particular of a surgical microscope system 100, comprising the system 110 and a surgical imaging device 120. The surgical microscope system 100 shown in Fig. 1b comprises a number of optional components, such as a base unit 105 (comprising the system 110) with a (rolling) stand, ocular displays 130b that are arranged at the imaging device 120, an auxiliary display 130a that may be arranged at the base unit, and the arm that holds the microscope 120 in place, and is coupled to the base unit and to the microscope 120. In Fig. 1b, the surgical imaging system 100 further comprises a microphone 140, an overview camera 150, and an OCT scanner 160.

In the proposed concept, the system 110 is used for the purpose of controlling spatially localized functionalities of the surgical imaging system 100 by tracking the position of a portion (e.g., a tip, blade, or tube opening) of a surgical tool, such as a scalpel, forceps, or a suction device. In particular, the system 110 is configured to obtain and process a stream of image data (e.g., a video stream or a sequence of images) from an imaging device 120, 150 of the surgical imaging system. For example, the system 110 may be configured to receive or read out the image data from the respective imaging device 120, 150 of the surgical imaging system. For example, the stream of image data may be obtained from the surgical imaging device 120, such as the surgical exoscope or surgical microscope, or from an overview camera 150 of the surgical imaging system. In this context, the overview camera 150 of the surgical imaging system is a separate, wide-field-of-view camera that provides a broader anatomical context around the surgical site. Unlike the main surgical imaging device 120, which delivers a high-magnification, detailed view through the eyepieces, the overview camera 150 displays a lower-magnification, wider perspective of the entire operating field to the surgical team.

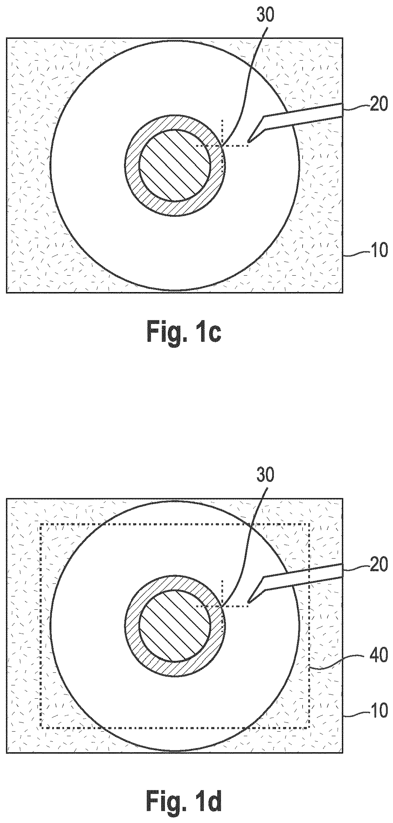

Regardless of the source of the stream of image data, it is processed to track the position of the portion of the surgical tool 20. Figs. 1c and 1d show examples of an overlay comprising a digital pointing indicator 30 superimposed on a view of a surgical site (i.e., an eye). In Figs. 1c and 1d, an example image frame of the stream of image data of a field of view of the surgical imaging device 120 is shown, in which the tip of a surgical tool 20 is visible. A similar image frame, albeit at a lower magnification, can be obtained from the optional overview camera 150. In both types of image frames, the surgical tool 20 is clearly visible. This means that the position of the tip, blade or tube opening can be determined visually. The system 110 is configured to process the stream of image data to determine and track a position of the portion of the surgical tool, e.g., relative to the surgical site, relative to the field of view of the surgical imaging device 120, or relative to the field of view shown in the stream of image data (which can be the field of view of the overview camera. This can be done using various mechanisms.

A first mechanism is based on the use of machine learning. In other words, machine learning models and/or algorithms may be used to determine the position of the portion of the tool, such as its tip. For example, given that a limited number of surgical tools (scalpel, forceps, scissors, retractor, hemostat, surgical drill, surgical saw, suction device, etc.) are being used during surgery, a machine learning model may be trained to predict the position of the portion of the surgical tool. In particular, pose estimation may be used for this purpose. In other words, the system may be configured to process the image data using a machine-learning model to estimate a pose of the surgical tool and to determine the position of the portion of the surgical tool based on the estimated pose of the surgical tool.

Pose estimation is a computer vision technique that determines the position and orientation of objects in 2D or 3D space from image data. In surgical applications, it involves detecting and tracking the spatial coordinates, rotation angles, and overall configuration of surgical instruments, anatomical structures, or other relevant objects within the field of view. For surgical imaging applications, pose estimation using machine learning typically involves deep neural networks. These models process the microscope's image data by identifying key features and landmarks of the target object. The network can be designed to output either direct pose parameters (position and orientation) or intermediate representations like keypoints that can be used to derive the pose.

Training such a machine-learning model requires a large dataset of labeled images showing the objects of interest from various angles, positions, and lighting conditions. The training data further include ground truth pose information for each image. This can be obtained through manual annotation, synthetic data generation, or using other sensing systems as a reference. The model learns to associate visual features with corresponding pose parameters through supervised learning, minimizing the difference between predicted and actual poses.

To determine the position of a specific portion of a surgical tool, such as a scalpel blade, the system can combine the pose estimation results with known object dimensions and imaging device parameters, such as zoom level and working distance. Using the overall pose as a starting point, the position of any point on the object can be calculated using geometric transformations. The system can compute the location of specific features, such as the scalpel blade tip or edges, as the position of the portion of the surgical tool with respect to pixel coordinates relative to the stream of image data, or relative to the surgical site if the working distance and zoom factor of the respective imaging device are known.

A second technique for determining the position of the portion of the surgical tool uses a 3D model of the surgical tool to determine the pose, i.e., position and orientation, of the surgical tool. In particular, the system may be configured to determine the position of the portion of the surgical tool by fitting a projection of a three-dimensional model of the surgical tool to the surgical tool shown in the image data. The projection of a three-dimensional model of a surgical tool onto the image data can be accomplished through a process called model-based tracking. By creating a virtual projection of the three-dimensional model of the tool and overlaying it onto the 2D microscope image, an optimization algorithm can be used to adjust the position and orientation of the projected model until it best matches the actual tool visible in the image. This fitting process typically involves reducing or minimizing the difference between the projected model's edges and the corresponding edges detected in the image data. The algorithm iteratively refines the tool's pose parameters (position and orientation) until the projected model closely aligns with the real tool's image. Once the projection is accurately fitted to the surgical tool in the image data, the position of the portion of the tool (such as a scalpel blade) can be determined.

In the proposed concept, the position of the portion of the surgical tool is not only determined once but is also tracked. Tracking the position of the portion of the surgical tool means determining its position multiple times to establish relative change and movement. In other words, the position of the surgical tool is determined multiple times, and the relative change in position is determined. In other words, the system 110 may be configured to determine a relative movement of the portion of the surgical tool.

In many cases, multiple surgical tools may be (partially) visible in the field of view at once. To determine which of the surgical tools is to be used for moving the digital pointing indicator, the surgeon can identify the surgical tool to be used by moving (e.g., shaking) the surgical tool after activating the digital pointing indicator. For example, the system 110 may be configured to, if multiple surgical tools are visible in the field of view of the surgical imaging device, determine the surgical tool being used to control the digital pointing indicator based on a movement (e.g., a shaking movement) of the surgical tool after activation of the digital pointing indicator. For example, the system 110 may be configured to use the determined positions of the surgical tool to detect the movement of the surgical tool, and to disregard the positions of other surgical tools for the purpose of determining the position of the digital pointing indicator. To let the surgeon know that the surgical tool has been successfully identified, the system 110 may be configured to provide visual or audio feedback (e.g., a confirmation sound or an outline around the surgical tool as part of the overlay) after determining the surgical tool being used to control the digital pointing indicator.

The system uses the change of the position of the portion of the surgical tool to determine the position of the digital pointing indicator 30, e.g., as shown in Figs. 1c and 1d. In other words, the system 110 is configured to determine, based on the change of the position of the portion of a surgical tool, the position of a digital pointing indicator 30. In particular, the position of the digital pointing indicator 30 (i.e., a cursor or pointer, such as the crosshair shown in Figs. 1c and 1d, which is to be superimposed over the field of view of the surgical imaging device 120) is determined relative to a field of view 10 of the surgical imaging device 120, with the field of view showing the surgical site. This does not (necessarily) mean that the pointing indicator is placed in the field of view at the position at which the tip of the surgical tool is shown. On the contrary, in the proposed concept, the digital pointing indicator 30 is moved based on the relative movement of the portion of the surgical tool, e.g., akin to a mouse/trackpad cursor. In other words, the system 110 may be configured to determine the position of the digital pointing indicator based on a relative movement of the portion of the surgical tool. For example, upon activation of the digital pointing indicator, the system 110 may be configured to move the digital pointing indicator 30 relative to the field of view 10 of the surgical imaging device 120 in line with the movement of the portion of the surgical tool. In particular, to avoid blocking the surgeon's view of the surgical site and the surgical tool, the system 110 may be configured to determine the position of the digital pointing indicator so that the digital pointing indicator 30 is (generally) offset from the portion of the surgical tool, as perceived by a user viewing the digital pointing indicator overlaid over the view of the surgical site by the surgical imaging device.

In most cases, spatially localized functionalities, i.e., functionalities that are to be performed at a specific location, such as a focusing functionality, a functionality for setting a marker, a functionality for changing the field of view, or a functionality for performing a measurement using a measurement device (such as the OCT scanner 160 shown in Fig. 1b), are more commonly applied at a location towards the center of the field of view than at a location at the periphery of the field of view, as the surgeon usually sets the field of view so that the relevant portion of the surgical site is at the center. To facilitate the placement of the digital pointing indicator, it may be restricted to the center of the field of view. For example, as shown in Fig. 1d, the system 110 may be configured to determine the position of the digital pointing indicator 30 so that the digital pointing indicator is constrained to a central portion 40 of the field of view, e.g., to a rectangular portion 40 of the field of view at the center of the field of view. For example, this central portion may cover at least 30% (or at least 40%, or at least 50% and/or at most 90% (and/or at most 80%, at most 70%) of the area of the field of view of the surgical imaging device.

In the proposed concept, the digital pointing indicator 30 is used to select a position at which the spatially localized functionality is to be triggered. For this purpose, as shown in Figs. 1c and 1d, the digital pointing indicator 30 is shown superimposed over the field of view 10 of the surgical imaging device 120, which the surgeon uses to perform the surgical procedure. This is done using an overlay that is to be overlaid over the field of view of the surgical imaging device 120. In this context, an overlay is a digital information layer that is shown on top of a digital view or optical view of the surgical site. The system 110 is configured to generate the overlay comprising the digital pointing indicator to be overlaid over the field of view of the surgical imaging device. For example, in the overlay, the digital pointing indicator 30 may be shown as a point, a circle, a mouse cursor or the like.

The system 110 is configured to provide the overlay with the digital pointing indicator 30 for a display device 130a, 130b of the surgical imaging system. The aim is to show the digital pointing indicator 30 superimposed over the view of the surgical site, as provided by the surgical imaging device 120. To achieve this, two cases can be distinguished. In a first case, if the view of the surgical site is a (non-digital) optical view, the overlay can be overlaid over the view by projecting the overlay onto a semi-transparent mirror or displaying the overlay on a semi-transparent display, with the mirror or display arranged in the optical path between the oculars and the objective of the surgical imaging device. In this case, the display device may be a projector or a semi-transparent display. In many cases, the surgical imaging device 120 may be a hybrid or fully digital surgical imaging device. Therefore, in a second case, the overlay may be overlaid over a digital view of the surgical site, as provided by an optical imaging sensor of the surgical imaging device 120. Accordingly, the system 110 may be configured to obtain image data of the surgical imaging device 120 (e.g., the stream of image data) and generate a (digital) view of a surgical site overlaid with the overlay based on the image data. The system 110 may be configured to provide the view of the surgical site with the overlay to the display device 130a, 130b of the surgical imaging system.

The digital pointing indicator 30 is shown overlaid over the field of view of the surgical imaging device 120 to enable the surgeon to select a location within the surgical site where a spatially localized functionality is to be performed. While selecting the location is performed by moving the digital pointing indicator relative to the field of view of the surgical imaging device 120, the spatially localized functionality is triggered at the position where the digital pointing indicator is shown using a voice command. Accordingly, the system 110 is configured to obtain a voice command of (e.g., from) a user of the surgical imaging system (e.g., via microphone 140), with the voice command comprising an instruction to perform a spatially localized functionality. For example, the system 110 may be configured to perform voice recognition or a speech-to-text algorithm or machine-learning model on the voice command, and use the result of the voice recognition or speech-to-text algorithm or machine-learning model to determine that and/or which spatially localized functionality is to be triggered. The user/surgeon may be provided with feedback that the voice command has been successfully recognized. Accordingly, the system may be configured to, after processing the voice command, include a visual indicator representing the voice command in the overlay, e.g., an icon or text representing the spatially localized functionality.

Some spatially localized functionalities can be applied to an area of the surgical site, as opposed to a single position at the surgical site. For example, an area can be selected by determining two positions of the digital pointing indicator corresponding to two corners of an area. For example, the system 110 may be configured to, if the voice command (e.g., a spatially localized functionality associated with the voice command) indicates that the spatially localized functionality is to be performed at an area, determine two positions of the digital pointing indicator relative to the field of view of the surgical imaging device of the surgical imaging system. These two positions may be treated as opposite corners of a rectangle defining the area. Thus, the system 110 may be configured to determine the area based on the two positions and based on an orientation of the field of view of the surgical imaging device.

The system 110 is configured to trigger performing the spatially localized functionality according to the position or positions (i.e., the area) of the digital pointing indicator relative to the field of view of a surgical imaging device (i.e., at the position of the surgical site at which the digital pointing indicator is shown in the field of view of the surgical imaging device) based on the voice command. For example, the system 110 may comprise a data storage of at least one trigger word or sentence associated with at least one spatially localized functionality, and to trigger the spatially localized functionality upon detection of a corresponding trigger word or sentence.

In the following, some concrete examples of the proposed concept are provided. The proposed concept introduces cursor-linked surgical imaging device (e.g., microscope) guidance and smart control, by providing a cursor-linked control system integrated into the surgical imaging system (e.g., surgical microscope system). This system enables surgeons to intuitively interact with the imaging device's functions through the movement of their surgical tools and voice commands.

The system tracks the position and movement of the surgical tool within the imaging device's field of view. This allows a cursor (digital pointing indicator) to move correspondingly. Surgeons can then use voice commands to perform functions, such as focusing by saying 'focus here' or initiating an Optical Coherence Tomography (OCT, an example of a measurement device 160, as shown in Fig. 1b, that is applied spatially localized at the surgical site) scan by saying "scan here", directly at the cursor's location.

The proposed concept eliminates the need for cumbersome manual controls, such as foot pedals, touch screens, or mouth controllers. By combining tool tracking and voice recognition, the system significantly streamlines the workflow, reduces cognitive load, and enhances precision. Unlike other approaches, this system allows for seamless, hands-free operation. This improves efficiency and enables surgeons to remain fully focused on the procedure.

The proposed concept integrates a cursor-linked control system with voice command functionality into a surgical microscope. This innovative integration enables intuitive interaction by tracking the movement of surgical tools within the imaging device's field of view and linking those movements to an on-screen cursor. Surgeons can utilize voice commands to direct the surgical imaging system (e.g., the surgical imaging device) to perform specific functions, such as focusing or scanning, at the cursor's location. By eliminating the need for manual navigation via foot pedals, touch screens, or mouth controllers, this system enhances workflow efficiency, streamlining operations and improving precision.

The proposed concept is based on tool tracking and cursor integration: a tracking mechanism (such as optical, infrared, or other motion-tracking technologies) is used to detect the position and movement of the surgical tool in real time. These detected movements are linked to a cursor visible within the imaging device's field of view, allowing the cursor to move along with the tool.

Additionally, voice command functionality is integrated through a voice recognition functionality that may accept commands such as "Focus here" to adjust the microscope's focus at the cursor's location or "scan here" to initiate an Optical Coherence Tomography (OCT) scan at the specified point (i.e., in a spatially localized manner). Additional commands enable other functions, such as brightness adjustment or image capture. For example, the system may process voice commands using a noise-filtering system to improve reliability in the operating room environment.

The proposed concept also offers hands-free and intuitive operation by combining tool tracking and voice commands to minimize physical interactions with manual controls, enabling hands-free operation. Its intuitive design allows surgeons to focus on surgical procedures while reducing cognitive and physical strain.

Integration with existing microscope systems is possible, as the system can be retrofitted onto existing surgical microscopes or exoscopes or integrated into new designs. It may connect with current OCT modules and focus adjustment mechanisms, thereby leveraging existing optical and imaging capabilities.

The functional workflow begins with initialization. For example, the cursor system may be activated when the microscope detects the surgeon's tool within its field of view. The tracking module may then start monitoring the tool's position and display a cursor at the corresponding location. During tool tracking and cursor movement, as the surgeon moves the tool, the cursor dynamically follows in real time. The cursor may be precisely calibrated to align with the tip (or other portion) of the surgical tool for accurate positioning.

The surgeon may issue commands such as "focus here" or "scan here" as voice commands. These are processed by the voice recognition functionality, which may trigger executing the requested function at the cursor's location.

In various examples, feedback and confirmation may be provided. The system may provide immediate feedback through visual or auditory cues to confirm successful execution of the command. For instance, a "focus" command may result in a sharp, clear image, while an "OCT scan" command may display the scan results immediately, e.g., in real time.

In the proposed concept, a surgical imaging system displays a cursor that moves precisely in sync with the surgeon's tool within the field of view, as the cursor movement is linked to the tool. Focus adjustments or OCT scanning may be activated through voice commands such as "focus here" or "scan here", through the use of voice-controlled commands. The proposed workflow enables hands-free operation by eliminating the need for manual controls like foot pedals, touch screens, or mouth controllers. Immediate (e.g., real-time) feedback, e.g., visual or auditory feedback, may be used to confirm actions linked to cursor-based operations. The proposed system integrates tool tracking and voice recognition with existing microscope components such as OCT modules or focus mechanisms, providing an effective system integration.

In the proposed concept, the system 110 may be used for the purpose of generating and providing an overlay to be superimposed over the digital or optical view of the surgical site. For example, if a digital view is used, it is generated using image data of the surgical imaging device 120, e.g., by performing image processing on the image data of the optical imaging sensor(s) of the surgical imaging device. In addition, the aforementioned overlay is overlaid over the digital view of the surgical site. Alternatively, that is, if the overlay is overlaid over an optical view of the surgical site, only the overlay may be provided to the display device. In both cases, that is, in the case of the digital view plus overlay or only the overlay, the respective visual data (digital view plus overlay or only the overlay) is provided to a display device, such as ocular displays 130b, a stereo head-mounted display, or a monitor 130a. This visual data may be created and provided to the display device of the surgical imaging system as part of a display signal. In other words, the system 110 may be configured to output a display signal to the display device 130a, 130b of the surgical or scientific imaging system, with the display signal comprising the overlay, and optionally the digital view of the surgical site. For example, the display signal may be a signal for driving (e.g., controlling) the respective display device. For example, the display signal may comprise video data and/or control instructions for driving the display. For example, the display signal may be provided via one of the one or more interfaces 112 of the system. Accordingly, the system 110 may comprise a video interface 112 that is suitable for providing the display signal to the display device 130a, 130b of the microscope system 100.

In the proposed surgical imaging system, optical imaging sensor(s) of the surgical imaging device 120 (or of an overview camera 150) are used to provide the aforementioned image data. Accordingly, the optical imaging sensor(s), which may be part of the imaging device 120 (e.g., of the microscope) and/or the overview camera 150 may be configured to generate the image data. For example, the optical imaging sensor(s) of the imaging device 120 or overview camera 150 may comprise or be an APS (Active Pixel Sensor)-based imaging sensor or a CCD (Charge-Coupled-Device)-based imaging sensor. For example, in APS-based imaging sensors, light is recorded at each pixel using a photodetector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the imaging sensor by circuitry of the imaging sensor to perform the imaging.

The one or more interfaces 112 of the system 110 may correspond to one or more inputs and/or outputs for receiving and/or transmitting information, which may be in digital (bit) values according to a specified code, within a module, between modules or between modules of different entities. For example, the one or more interfaces 112 may comprise interface circuitry configured to receive and/or transmit information. The one or more processors 114 of the system 110 may be implemented using one or more processing units, one or more processing devices, any means for processing, such as a processor, a computer or a programmable hardware component being operable with accordingly adapted software. In other words, the described function of the one or more processors 114 may as well be implemented in software, which is then executed on one or more programmable hardware components. Such hardware components may comprise a general-purpose processor, a Digital Signal Processor (DSP), a micro-controller, etc. The one or more storage devices 116 of the system 110 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

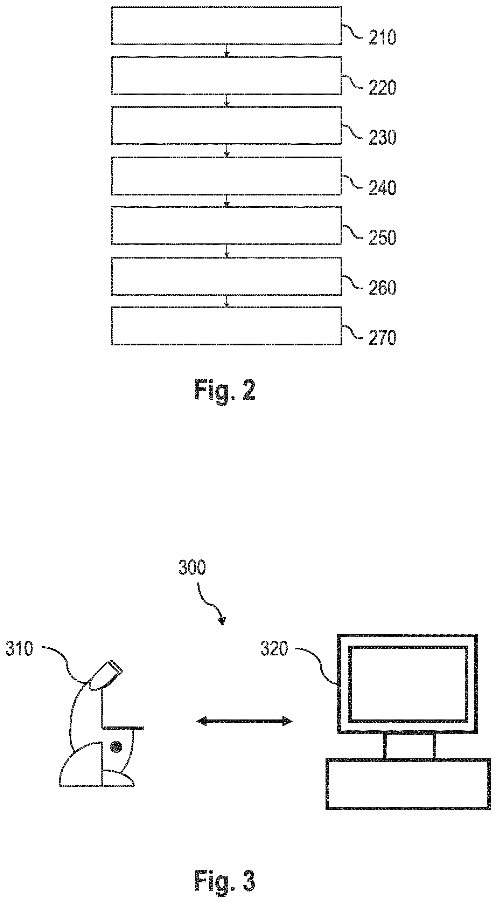

More details and aspects of the system 110 and of the surgical imaging system 100 are mentioned in connection with the proposed concept or with one or more examples described above or below (e.g., Figs. 2 or 3). The system 110 and the surgical imaging system 100 may comprise one or more additional optional features corresponding to one or more aspects of the proposed concept or with one or more examples described above or below.

Fig. 2 shows a flow chart of an example of a corresponding method for a surgical imaging system, such as the surgical imaging system 100 of Fig. 1b. Features discussed in connection with the surgical imaging system 100 and the system 110 of Figs. 1a to 1d may likewise be included in the corresponding method of Fig. 2. The method comprises obtaining 210 a stream of image data of an imaging device 120, 150 of the surgical imaging system. The method comprises processing 220 the stream of image data to track a position of a portion of a surgical tool 20. The method comprises determining 230, based on a change in the position of the portion of a surgical tool, a position of a digital pointing indicator 30 relative to a field of view 10 of a surgical imaging device 120 of the surgical imaging system showing a surgical site. The method comprises generating 240 an overlay comprising the digital pointing indicator to be overlaid over the field of view of the surgical imaging device. The method comprises providing 250 the overlay with the digital pointing indicator for a display device 130a, 130b of the surgical imaging system. The method comprises obtaining 260 a voice command of a user of the surgical imaging system. The voice command comprises an instruction to perform a spatially localized functionality. The method comprises triggering 270 performing the spatially localized functionality according to the position of the digital pointing indicator relative to the field of view of a surgical imaging device based on the voice command.

More details and aspects of the method are mentioned in connection with the proposed concept or one or more examples described above or below (e.g., Figs. 1a to 1d, or 3). The method may comprise one or more additional optional features corresponding to one or more aspects of the proposed concept or one or more examples described above or below.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to a surgical imaging device comprising a system as described in connection with one or more of the Figs. 1a to 2. Alternatively, a surgical imaging device may be part of or connected to a system as described in connection with one or more of the Figs. 1a to 2. Fig. 3 shows a schematic illustration of a system 300 configured to perform a method described herein. The system 300 comprises a surgical imaging device 310 and a computer system 320. The surgical imaging device 310 is configured to take images and is connected to the computer system 320. The computer system 320 is configured to execute at least a part of a method described herein. The computer system 320 may be configured to execute a machine learning algorithm. The computer system 320 and surgical imaging device 310 may be separate entities but can also be integrated together in one common housing. The computer system 320 may be part of a central processing system of the surgical imaging device 310 and/or the computer system 320 may be part of a subcomponent of the surgical imaging device 310, such as a sensor, an actor, a camera or an illumination unit, etc. of the surgical imaging device 310.

The computer system 320 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 320 may comprise any circuit or combination of circuits. In one embodiment, the computer system 320 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a surgical imaging device or a surgical imaging device component (e.g. camera) or any other type of processor or processing circuit. Other types of circuits that may be included in the computer system 320 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 320 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 320 may also include a display device, one or more speakers, and a keyboard and/or controller, which can include a mouse, trackball, touch screen, voice-recognition device, or any other device that permits a system user to input information into and receive information from the computer system 320.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all the functionalities of the methods described herein. In some embodiments, a field programmable gate array may cooperate with a microprocessor to perform one of the methods described herein. Generally, the methods are preferably performed by any hardware apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of reference Signs

- 10:: Field of view
- 110:: System
- 112:: Interface(s)
- 114:: Processor(s)
- 116:: Storage device(s)
- 120:: Surgical imaging device
- 130a:: Auxiliary display
- 130b:: Ocular display(s)
- 140:: Microphone
- 150:: Overview camera
- 160:: OCT scanner
- 20:: Surgical tool
- 30:: Digital pointing indicator
- 40:: Central portion (of the field of view)
- 100:: Surgical imaging system
- 105:: Base unit
- 210:: Obtaining a stream of image data
- 220:: Processing the stream of image data to track a position of a portion of a surgical tool
- 230:: Determining a position of a digital pointing indicator
- 240:: Generating an overlay comprising the digital pointing indicator
- 250:: Providing the overlay with the digital pointing indicator for a display device
- 260:: Obtaining a voice command
- 270:: Triggering performing the spatially localized functionality
- 300:: System
- 310:: Imaging device
- 320:: Computer system

## Claims

1. A system (110) for a surgical imaging system (100), the system comprising one or more processors and one or more storage devices, wherein the system is configured to:
obtain a stream of image data of an imaging device (120, 150) of the surgical imaging system;
process the stream of image data to track a position of a portion of a surgical tool (20);
determine, based on a change in the position of the portion of the surgical tool, a position of a digital pointing indicator (30) relative to a field of view (10) of a surgical imaging device (120) of the surgical imaging system showing a surgical site;
generate an overlay comprising the digital pointing indicator to be overlaid over the field of view of the surgical imaging device;
provide the overlay with the digital pointing indicator for a display device (130a, 130b) of the surgical imaging system;
obtain a voice command of a user of the surgical imaging system, the voice command comprising an instruction to perform a spatially localized functionality; and
trigger performing the spatially localized functionality according to the position of the digital pointing indicator relative to the field of view of the surgical imaging device based on the voice command.

2. The system according to claim 1, wherein the system is configured to obtain image data of the surgical imaging device (120), generate a view of the surgical site overlaid with the overlay and provide the view of the surgical site with the overlay to the display device (130a, 130b) of the surgical imaging system.

3. The system according to one of the claims 1 or 2, wherein the system is configured to obtain the stream of image data from the surgical imaging device (120) of the surgical imaging system.

4. The system according to one of the claims 1 or 2, wherein the system is configured to obtain the stream of image data from an overview camera (150) of the surgical imaging system.

5. The system according to one of the claims 1 to 4, wherein the spatially localized functionality is one of a focusing functionality, a functionality for setting a marker, a functionality for changing the field of view, or a functionality for performing a measurement using a measurement device (160).

6. The system according to one of the claims 1 to 5, wherein the system is configured to, if the voice command indicates that the spatially localized functionality is to be performed on an area, determine two positions of the digital pointing indicator relative to the field of view of a surgical imaging device of the surgical imaging system, determine the area based on the two positions and based on an orientation of the field of view of the surgical imaging device, and trigger performing the spatially localized functionality on the area.

7. The system according to one of the claims 1 to 6, wherein the system is configured to determine the position of the digital pointing indicator so that the digital pointing indicator (30) is offset from the portion of the surgical tool (120), as perceived by a user viewing the digital pointing indicator overlaid over the view of the surgical site by the surgical imaging device.

8. The system according to one of the claims 1 to 7, wherein the system is configured to determine the position of the digital pointing indicator so that the digital pointing indicator is constrained to a central portion (40) of the field of view of the surgical imaging device.

9. The system according to claim 8, wherein the system is configured to determine the position of the digital pointing indicator so that the digital pointing indicator is constrained to a rectangular portion (40) of the field of view at the center of the field of view of the surgical imaging device.

10. The system according to one of the claims 8 or 9, wherein the central portion of the field of view covers at least 30% and/or at most 90% of an area of the field of view of the surgical imaging device.

11. The system according to one of the claims 1 to 10, wherein the system is configured to determine the position of the digital pointing indicator based on a relative movement of the portion of the surgical tool.

12. The system according to one of the claims 1 to 11, wherein the system is configured to, if multiple surgical tools are visible in the field of view of the surgical imaging device, determine the surgical tool being used to control the digital pointing indicator based on a movement of the surgical tool after activation of the digital pointing indicator,
and/or wherein the system is configured to, after processing the voice command, include a visual indicator representing the voice command in the overlay,
and/or wherein the system is configured to determine the position of one of a tip, a blade, or a tube opening of the surgical tool.

13. A surgical imaging system (100) comprising the system according to one of the claims 1 to 12.

14. A method for a surgical imaging system (100), the method comprising:
obtaining a stream of image data of an imaging device (120, 150) of the surgical imaging system;
processing the stream of image data to track a position of a portion of a surgical tool (20);
determining, based on a change in the position of the portion of the surgical tool, a position of a digital pointing indicator (30) relative to a field of view (10) of a surgical imaging device (120) of the surgical imaging system showing a surgical site;
generating an overlay comprising the digital pointing indicator to be overlaid over the field of view of the surgical imaging device;
providing the overlay with the digital pointing indicator for a display device (130a, 130b) of the surgical imaging system;
obtaining a voice command of a user of the surgical imaging system, the voice command comprising an instruction to perform a spatially localized functionality; and
triggering performing the spatially localized functionality according to the position of the digital pointing indicator relative to the field of view of the surgical imaging device based on the voice command.

15. A computer program having a program code for performing the method according to claim 14 when the program is executed on a processor.
